# EUROPÄISCHE PATENTSCHRIFT

(11) **EP 2 996 776 B1**
(45) Veröffentlichungstag und Bekanntmachung des Hinweises auf die Patenterteilung: **24.05.2017**
(21) Anmeldenummer: 14701760.2
(22) Anmeldetag: 29.01.2014
(51) Int. Cl.: A61Q 5/08, A61Q 5/10, A61K 8/73, A61K 8/81, A61K 8/23, A61K 8/22, A61K 8/898, A61K 8/891

(54) **POLYMERHALTIGE TRANSPARENTE BLONDIERMITTEL MIT PROTEINEN UND/ODER SILIKONÖLEN**
POLYMER-CONTAINING TRANSPARENT BLEACHING COMPOSITIONS WITH PROTEINS AND/OR SILICONE OILS
PRODUIT DE DÉCOLORATION TRANSPARENT CONTENANT DES POLYMÈRES AVEC PROTÉINES ET/OU HUILES DE SILICONE

(30) Priorität: 16.05.2013 DE 102013209100
(43) Veröffentlichungstag der Anmeldung: 23.03.2016
(73) Patentinhaber: Henkel AG & Co. KGaA, 40589 Düsseldorf (DE)
(72) Erfinder: ANDERHEGGEN, Bernd, 41189 Mönchengladbach (DE); JANSSEN, Frank, 51103 Köln (DE)
(86) Internationale Anmeldenummer: PCT/EP2014/051681
(87) Internationale Veröffentlichungsnummer: WO 2014/183881

(56) Entgegenhaltungen:
- EP-A1- 2 191 812
- WO-A1-2005/067874
- DE-A1-102010 042 252

## Beschreibung

Die vorliegende Erfindung betrifft Mittel zur oxidativen Farbveränderung im Bereich der Kosmetik, die sich besonders zum Aufhellen keratinischer Fasern, insbesondere menschlicher Haare, eignen.

Die in Blondiermitteln enthaltenen Oxidationsmittel sind in der Lage, die Haarfaser durch die oxidative Zerstörung des haareigenen Farbstoffes Melanin aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein, für das Erzielen eines stärkeren Blondiereffektes wird üblicherweise eine Mischung aus Wasserstoffperoxid und Peroxodisulfatsalzen und/oder Peroxomonosulfatsalzen eingesetzt.

Aus Stabilitätsgründen werden handelsübliche Blondiermittel gewöhnlich in zwei getrennt voneinander verpackten Zubereitungen angeboten, die unmittelbar vor der Anwendung zu einer fertigen Anwendungszubereitung vermischt werden. Üblicherweise bestehen handelsübliche Blondiermittel aus einer flüssigen Oxidationsmittelzubereitung und einem Pulver, das feste Oxidationsmittel enthält. Produkte mit weiteren Komponenten werden ebenfalls im Handel angeboten.

WO 2005/067874 A1 beschreibt Blondiermittel, welche eine Mischung aus einem Oxidationsmittel, mindestens einem Stabilisator, mindestens einem Polymerverdicker aus synthetischen Polymeren und Alkalimagnesiumsilikaten und Wasser oder ein wässriges Lösungsmittel enthalten. Als wünschenswerte Eigenschaften des Mittels gemäß dieser Erfindung sind "Transparenz" und "angedickte Konsistenz" angegeben.

Die DE 10 2010 042 252 A1 offenbart Mittel zum Aufhellen keratinischer Fasern, welche mindestens zwei getrennt voneinander verpackte Zubereitungen (A) und (B) sowie gegebenenfalls eine weitere getrennt von (A) und (B) verpackte Zubereitung (C) enthalten, die unmittelbar vor der Anwendung zu einer Anwendungsmischung vermischt werden, wobei die Zubereitungen (A) mindestens ein Persulfat enthalten und die Zubereitungen (B) fließfähig sind und mindestens ein Oxidationsmittel enthalten und wobei Zubereitungen (B) und/oder Zubereitungen (C) weiterhin mindestens ein natürliches Polymer enthalten.

Die oxidative Behandlung keratinischer Fasern führt nicht nur zu dem gewünschten Aufhellergebnis, sie belastet die Fasern und kann die Faserstruktur im schlimmsten falle schädigen. Um diese negativen Auswirkungen zu minimieren, werden in herkömmlichen nicht erfindungsgemäßen nicht transparenten Blondiermitteln Pflegestoffe eingesetzt, die reparierende und konditionierende Wirkungen besitzen. Viele der üblicherweise eingesetzten Pflegestoffe führen in transparenten Blondiermitteln aber zu einem Verlust oder zu einer deutlichen Beeinträchtigung der Transparenz, was den Anwendungsvorteil dieser Mittel konterkariert. Andere Pflegestoffe schwächen die Blondierleistung, weshalb deren Einsatz kontraindiziert ist.

Aufgabe der vorliegenden Erfindung war es, die Eigenschaften von Blondiermitteln in Bezug auf ihre pflegenden Eigenschaften zu verbessern, ohne ihre Leistung zu beeinträchtigen.

Es hat sich gezeigt, dass sich bestimmte Verdicker gut in Blondierpulver einarbeiten lassen, welche bei ihrer Vermischung mit Oxidationsmittelzubereitungen eine fließfähige Zubereitung ergeben lassen, welche eine gute Homogenisierung der beiden Komponenten zu einer fertig vermischten Anwendungsmischung gestattet. In dieser speziellen Matrix führen bestimmte Proteine in Kombination mit/ohne Silikonöl zu permanenten Pflegeeffekten, darüber hinaus wird die Blondierleistung verstärkt. Gegenstand der vorliegenden Erfindung sind in einer ersten Ausführungsform Mittel zum Aufhellen keratinischer Fasern, enthaltend mindestens zwei getrennt voneinander verpackte Zubereitungen (A) und (B) sowie gegebenenfalls eine weitere getrennt von (A) und (B) verpackte Zubereitung (C), die unmittelbar vor der Anwendung zu einer Anwendungsmischung vermischt werden, wobei
i. Zubereitung (A) mindestens ein Persulfat, mindestens ein Acrylatpolymer und Carboxymethylcellulose enthält und
ii. Zubereitung (B) fließfähig ist und mindestens ein Oxidationsmittel enthält,
und Zubereitung (A) - bezogen auf ihr Gewicht -
a1) 0 bis 3 Gew.-% Keratinhydrolysat(e) und/oder
a2) 0 bis 5 Gew.-% Silikonöl(e) enthält,
mit der Maßgabe, dass die Zubereitung (A) - bezogen auf ihr Gewicht - 0,1 bis 6 Gew.-% Verbindung(en) aus den Gruppen a1) und a2) enthält.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Die Zubereitungen (A) sind bevorzugt pulverförmig. Dabei können Pulver aus festen Bestandteilen mit unterschiedlichen Korngrößen eingesetzt werden. Üblicherweise kann es bevorzugt sein, wenn die Pulver jedoch eine möglichst homogene Korngröße aufweisen, insbesondere um eine einheitliche Dispersion bzw. Auflösung der Pulver in den Zubereitungen (B) zu erleichtern.

Die Zubereitungen (A) können die Wirkstoffe in einem festen kosmetischen Träger enthalten. Ein fester kosmetischer Träger kann Salze der Kieselsäure, insbesondere Salze der Silicate und Metasilicate mit Ammonium, Alkalimetallen sowie Erdalkalimetallen enthalten. Insbesondere Metasilicate, die sich gemäß Formel (SiO₂)ₙ(M₂O)ₘ, wobei M für ein Ammoniumion, ein Alkalimetall oder ein halbes Stöchiometrieäquivalents eines Erdalkalimetalls steht, durch das Verhältnis zwischen n und m von ≤ 1 auszeichnen und sich als kettenförmige polymere Strukturen des Anions [SiO₃]²⁻ auffassen lassen, können bevorzugt eingesetzt werden. Natriummetasilicat der Formel [Na₂SiO₃]_{∞}, ist dabei besonders bevorzugt. Erfindungsgemäß gleichfalls bevorzugt sind solche Silicate, die aus einem Silicat der Formel (SiO₂)ₙ(Na₂O)ₘ(K₂O)ₚ gebildet werden, wobei n für eine positive rationale Zahl und m und p unabhängig voneinander für eine positive rationale Zahl oder für 0 stehen, mit den Maßgaben, dass mindestens einer der Parameter m oder p von 0 verschieden ist und das Verhältnis zwischen n und der Summe aus m und p zwischen 2:1 und 4:1 liegt.

Weiterhin können die festen kosmetischen Träger so genannte Rieselhilfen enthalten, die ein Verklumpen oder Verbacken der Pulver-Bestandteile verhindern sollen. Als solche Rieselhilfen kommen bevorzugt wasserunlösliche, hydrophobierende oder Feuchtigkeit adsorbierende Pulver von Kieselgur, pyrogenen Kieselsäuren, Calciumphosphat, Calciumsilicaten, Aluminiumoxid, Magnesiumoxid, Magnesiumcarbonat, Zinkoxid, Stearaten, Fettaminen und dergleichen in Frage. Schließlich können die festen kosmetischen Träger noch zusätzlich ein Entstaubungsmittel enthalten, die die Staubbildung der pulverförmigen Bestandteile verhindert. Hierzu können insbesondere inerte Öle eingesetzt werden. Bevorzugt enthalten die festen, kosmetischen Träger als Entstaubungsmittel Esteröle oder Mineralöle, bevorzugt Kohlenwasseröle, wie flüssiges Paraffinöl.

Als ersten wesentlichen Inhaltsstoff enthält die Zubereitung (A) mindestens ein Persulfat.

Erfindungsgemäß geeignete Persulfate sind anorganische Peroxoverbindungen. Diese sind bevorzugt ausgewählt aus Ammoniumperoxodisulfat, Alkalimetallperoxodisulfaten, Ammoniumperoxomonosulfat, Alkalimetallperoxomonosulfaten, Alkalimetallperoxodiphosphaten und/oder Erdalkalimetallperoxiden.

Besonders bevorzugt sind Ammoniumperoxodisulfat und/oder Alkalimetallperoxodisulfate.

In einer bevorzugten Ausführungsform der vorliegenden Erfindung enthält Zubereitung (A) als Persulfat mindestens ein Peroxodisulfat-Salz, insbesondere ausgewählt aus Ammoniumperoxodisulfat und/oder Kaliumperoxodisulfat und/oder Natriumperoxodisulfat.

Weiterhin hat es sich bei den Arbeiten zur vorliegenden Erfindung als besonders bevorzugt erwiesen, wenn die Zubereitungen (A) mindestens zwei verschiedene Peroxodisulfate enthalten. Bevorzugte Peroxodisulfat-Salze sind dabei Kombinationen von Ammoniumperoxodisulfat mit Kaliumperoxodisulfat und/oder Natriumperoxodisulfat.

Die Zubereitungen (A) enthalten vorzugsweise Persulfat-Salze in einer Menge von 0,1 bis 80 Gew.-%, bevorzugt von 2 bis 50 Gew.-%, besonders bevorzugt von 3 bis 30 Gew.-% und insbesondere bevorzugt 5 bis 15 Gew.-% explizit 5, 6, 7, 8, 9, 10, 11, 12, 13, 14 oder 15 Gew.-% jeweils bezogen auf das Gesamtgewicht des Mittels.

Als weiteren wesentlichen Inhaltsstoff enthält die Zubereitung (A) mindestens ein Acrylatpolymer und Carboxymethylcellulose.

Acrylatpolymere im Sinne der vorliegenden Anmeldung sind Polymere, die Monomereinhaiten aus Acrylsäure und/oder Methacrylsäure und ihren jeweiligen Derivaten beinhalten. Eingesetzt werden können beispielsweise Homopolymere wie Polyacrylsäure, Polymethacrylsäure sowie ihre jeweiligen Salze und/oder Ester.

Solche Hompolymeren enthalten ausschließlich Struktureinheiten der Formel (I) 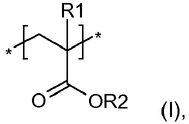 in der R1 für -H oder -CH₃ und R2 für -H oder -CH₃ oder -CH₂CH₃ oder -CH₂CH₂CH₃ oder - CH(CH₃)₂ steht. Wenn R1 für-H steht, handelt es sich bei den zugrundeliegenden Monomeren der Formel (I) um Acrylsäure oder Acrylsäureester, bei R1 = -CH₃ handelt es sich bei den zugrundeliegenden Monomeren der Formel (I) um Methacrylsäure oder Methacrylsäureester.
R2 kann auch ganz oder teilweise für Na⁺, K⁺, NH₄⁺ oder den n-ten Teil eines n-fach geladenen Kations stehen, in diesem Fall sind die Hompolymere die (teil-)neutraliserten Polysäuren.
Aber auch Copolymere, die weitere Monomere aufweisen, sind erfindungsgemäß einsetzbar. Bevorzugte Acrylat-Copolymere sind beispielsweise Polyacrylsäure und/oder Copolymere von Methacrylsäure mit Acrylamidopropansulfonsäure und/oder Copolymere von Acrylsäure mit Methacrylsäure und Acrylsäureestern und/oder Copolymere von Acrylsäure mit Methacrylsäure mit Acrylsäureestern und Methacrylsäureestern und/oder Copolymere von Acrylsäureestern mit Methacrylsäure.

Na-Carboxymethylcellulose ist das Natriumsalz des Glycolsäureethers der Cellulose. Na-Carboxymethylcellulose wird technisch hergestellt durch Umsetzung von Alkalicellulose mit Monochloressigsäure bzw. deren Natriumsalz. Der Substitutionsgrad gibt an, wie viele der Reste R in der o.g. Formel für Wasserstoffatome bzw. -CH₂-COONa-Gruppierungen stehen. Ein Substitutionsgrad von 1 bedeutet dabei, dass einer der sechs Reste R pro wiederkehrender Formeleinheit für eine -CH₂-COONa-Gruppierung steht, während die restlichen fünf Reste -H sind.

Es ist erfindungsgemäß bevorzugt, die Na-CMC in engeren Mengenbereichen einzusetzen. Besonders bevorzugt sind - bezogen auf das Gewicht der Zubereitung (A) - Mengen von 0,25 bis 4,0 Gew.-%, vorzugsweise von 0,5 bis 3,0 Gew.-%, besonders bevorzugt von 0,75 bis 2,5 und insbesondere von 1,0 bis 1,75 Gew.-%.

Unabhängig von der Menge der eingesetzten Na-CMC sind Carboxymethycellulosen bevorzugt, die einen Substitutionsgrad von 0,75 bis 0,85 aufweisen.

Erfindungsgemäß enthält Zubereitung (A) - bezogen auf ihr Gewicht -
a1) 0 bis 3 Gew.-% Keratinhydrolysat(e) und/oder
a2) 0 bis 5 Gew.-% Silikonöl(e),
mit der Maßgabe, dass die Zubereitung (A) - bezogen auf ihr Gewicht - 0,1 bis 6 Gew.-% Verbindung(en) aus den Gruppen a1) und a2) enthält.

Erfindungsgemäße Mittel können demnach in der Zubereitung (A) ausschließlich Keratinhydrolysat(e) enthalten - dann in Mengen von 0,1 bis 3 Gew.-%, oder ausschließlich Silikonöl(e) - dann in Mengen von 0,1 bis 5 Gew.-% oder sowohl Keratinhydrolysat(e) als auch Silikonöl(e) - dann in Gesamtmengen beider Inhaltsstoffe von 0,1 bis 6 Gew.-%, jeweils bezogen auf das Gewicht der Zubereitung (A).

Erfindungsgemäß bevorzugte Mittel sind dadurch gekennzeichnet, dass die Zubereitung (A) 0,01 bis 1 Gew.-%, vorzugsweise 0,05 bis 0,75 Gew.-%, weiter bevorzugt 0,1 bis 0,5 Gew.-%, besonders bevorzugt 0,15 bis 0,4 Gew.-% und insbesondere 0,2 bis 0,3 Gew.-% Hydrolysat(e) mit Molmassen von 400 bis 1200 Dalton, gewonnen aus dem Kortex und/oder der Cuticula von keratinischen Fasern, enthält.

Weitere erfindungsgemäß bevorzugte Mittel sind dadurch gekennzeichnet, dass die Zubereitung (A) 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% Silikonöl(e) aus der Gruppe der Verbindungen mit der INCI-Bezeichnung Dimethicone enthält.

Dimethicone können sowohl linear als auch verzweigt als auch cyclisch oder cyclisch und verzweigt sein. Lineare Dimethicone können durch die folgende Strukturformel (Si1) dargestellt werden:

(SiR¹₃) - O - (SiR²₂ - O -)ₓ - (SiR¹₃) (Si1)

Verzweigte Dimethicone können durch die Strukturformel (Si1.1) dargestellt werden:

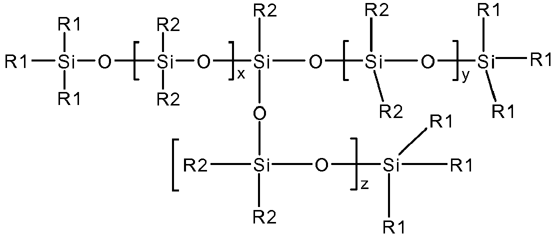

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, -CH₂CH₂OCH₂-, -OCH₂CH₂-, -OCH₂ CH₂CH₂-, -CH₂CH(CH₃)C(O)OCH₂-, -(CH₂)₃CC(O)OCH₂CH₂-, -C₆H₄C₆H₄-, -C₆H₄CH₂C₆H₄-; und -(CH ₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs. Höchst bevorzugt sind Viskositäten um den Bereich von etwa 60.000 cPs herum. Das Wort "etwa" definiert dabei eine dem Fachmann bei technisch hergestellten Produkten übliche Abweichung von dem genannten Wert im Anschluss an das Wort "etwa". Beispielhaft sei hier auf das Produkt "Dow Corning 200 mit 60000cSt" verwiesen.

Selbstverständlich umfasst die erfindungsgemäße Lehre auch, dass die Dimethicone bereits als Emulsion vorliegen können.

Wenn die Dimethicone als Emulsion verwendet werden, dann beträgt die Tröpfchengröße der emulgierten Teilchen erfindungsgemäß 0,01µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt.

Besonders bevorzugte erfindungsgemäße Mittel sind dadurch gekennzeichnet, dass die Zubereitung (A) 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% mindestens eines Silikons der Formel (Si-I)

(CH₃)₃Si-[O-Si(CH₃)₂]ₓ-O-Si(CH₃)₃ (Si-I),

enthalten, in der x für eine Zahl von 0 bis 100, vorzugsweise von 0 bis 50, weiter bevorzugt von 0 bis 20 und insbesondere 0 bis 10, steht.

Weitere erfindungsgemäß bevorzugte Mittel sind dadurch gekennzeichnet, dass die Zubereitung (A) 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% Silikonöl(e) aus der Gruppe der Verbindungen mit der INCI-Bezeichnung Amodimethicone enthält, wobei Verbindungen mit den INCI-Bezeichnungen Silicone Quaternium-1, Silicone Quaternium-2, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, Silicone Quaternium-16, Silicone Quaternium-17, Silicone Quaternium-18, Silicone Quaternium-20, Silicone Quaternium-21 oder Silicone Quaternium-22 bevorzugt sind.

Es hat sich gezeigt, dass insbesondere 4-Morpholinomethyl-substituierte Silikone zu besonders guten Pflegeergebnissen führen, ohne die Transparenz der Mittel zu beeinträchtigen. Besonders bevorzugte erfindungsgemäße Mittel sind dadurch gekennzeichnet, dass die Zubereitung (A) 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% Silikonöl(e) der Formel (I) enthält, 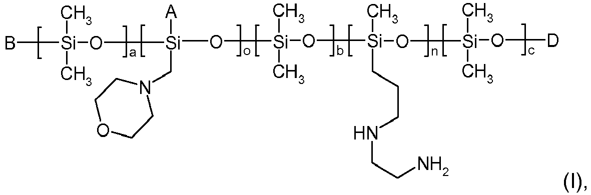 in der
A für eine über ein -O- gebundene Struktureinheit (I), (II) oder (III) 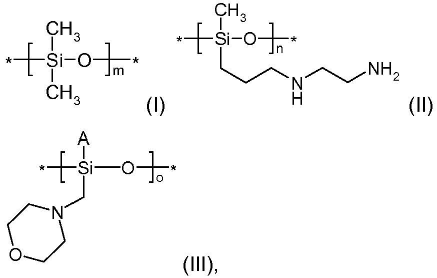 oder einen über ein -O- gebundenen oligomeren oder polymeren Rest enthaltend Struktureinheiten der Formeln (I), (II) oder (III) oder die Hälfte eines verbindenden O-Atoms zu einer Struktureinheit (III) oder für -OH steht,
   * für eine Bindung zu einer der Struktureinheiten (I), (II) oder (III) oder für eine Endgruppe B (Si-gebunden) oder D (O-gebunden) steht,
B für eine Gruppe -OH, -O-Si(CH₃)₃,-O-Si(CH₃)₂OH, -O-Si(CH₃)₂OCH₃ steht,
D für eine Gruppe -H, -Si(CH₃)₃,-Si(CH₃)₂OH, -Si(CH₃)₂OCH₃ steht,
a, b und c für ganze Zahlen zwischen 0 und 1000 stehen, mit der Maßgabe a + b + c > 0
m, n und o für ganze Zahlen zwischen 1 und 1000 stehen.

Solche bevorzugten Mittel enthalten in der Zubereitung (A) 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% mindestens eines 4-morpholinomethyl-substituierten Silikons der Strukturformel (I). Diese verdeutlicht, dass die Siloxangruppen n und o nicht zwingend direkt an eine Endgruppierung B bzw. D gebunden sein müssen. Vielmehr gilt in bevorzugten Formeln (I) a > 0 oder b > 0 und in besonders bevorzugten Formeln (I) a > 0 und b > 0, d.h. die terminale Gruppierung B bzw. D ist vorzugsweise an eine Dimethylsiloxy-Gruppierung gebunden. Auch in Formel (I) sind die Siloxaneinheiten a, b, c, n und o vorzugsweise statistisch verteilt.

Die durch Formel (I) dargestellten erfindungsgemäß eingesetzten Silikone können beidseitig trimethylsilyl-terminiert sein (D = -Si(CH₃)₃, B = -O-Si(CH₃)₃), sie können aber auch ein- oder zweiseitig dimethylsilylhydroxy- oder dimethylsilylmethoxy-terminiert sein. Im Rahmen der vorliegenden Erfindung besonders bevorzugt eingesetzte Silikone weisen mindestens eine endständige Dimethylsilylhydroxy-Gruppe auf, d.h. sind ausgewählt aus Silikonen, in denen

| | | |
|---|---|---|
| B = -O-Si(CH₃)₂OH | und | D = -Si(CH₃)₃ |
| B = -O-Si(CH₃)₂OH | und | D = -Si(CH₃)₂OH |
| B = -O-Si(CH₃)₂OH | und | D = -Si(CH₃)₂OCH₃ |
| B = -O-Si(CH₃)₃ | und | D = -Si(CH₃)₂OH |
| B = -O-Si(CH₃)₂OCH₃ | und | D = -Si(CH₃)₂OH |

bedeutet. Diese Silikone führen zu exorbitanten Verbesserungen der Haareigenschaften der mit den erfindungsgemäßen Mitteln behandelten Haare, insbesondere zu einer gravierenden Verringerung des Kontaktwinkels.

Die Struktureinheiten der Formeln (I), (II) und (III) können statistisch verteilt im Molekül vorliegen, die erfindungsgemäß eingesetzten Silikone können aber auch Blockcopolymere aus Blöcken der einzelnen Struktureinheiten sein, wobei die Blöcke wiederum statistisch verteilt vorliegen können.

Der * an den freien Valenzen der Struktureinheiten (I), (II) oder (III) steht dabei für eine Bindung zu einer der Struktureinheiten (I), (II) oder (III) oder für eine Endgruppe B (Si-gebunden) oder D (O-gebunden). In Formel (I) kann der Rest A
- für eine über ein -O- gebundene Struktureinheit (I), (II) oder (III) oder
- einen über ein -O- gebundenen oligomeren oder polymeren Rest enthaltend Struktureinheiten der Formeln (I), (II) oder (III)
- oder die Hälfte eines verbindenden O-Atoms zu einer Struktureinheit (III) oder für -OH stehen.

Damit wird Formel (I) präzisiert zu einer der Formeln (la), (Ib), (Ic), (Id), (Ie) oder (If):

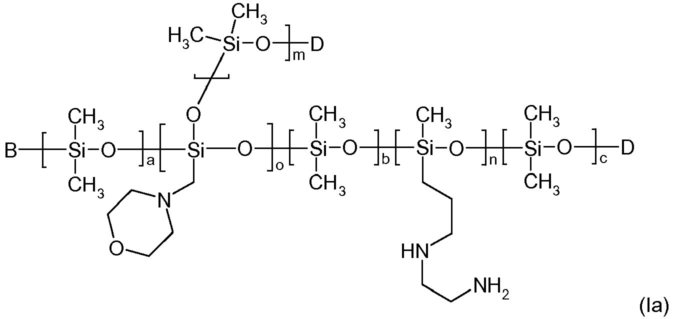

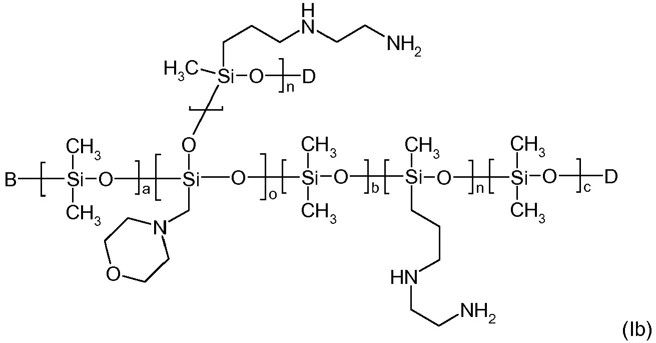

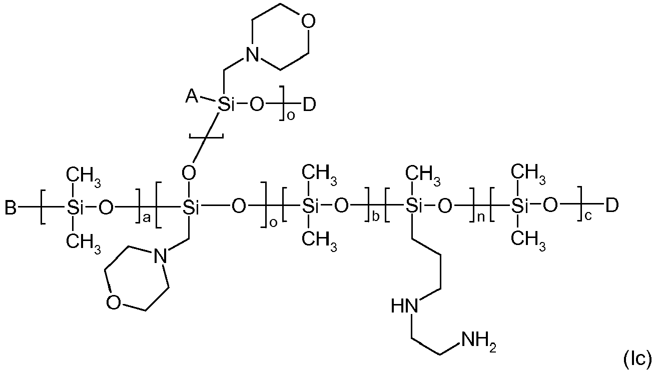

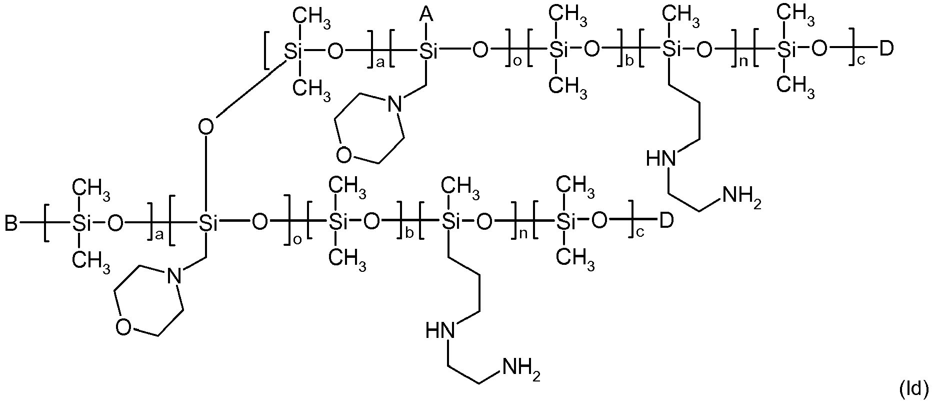

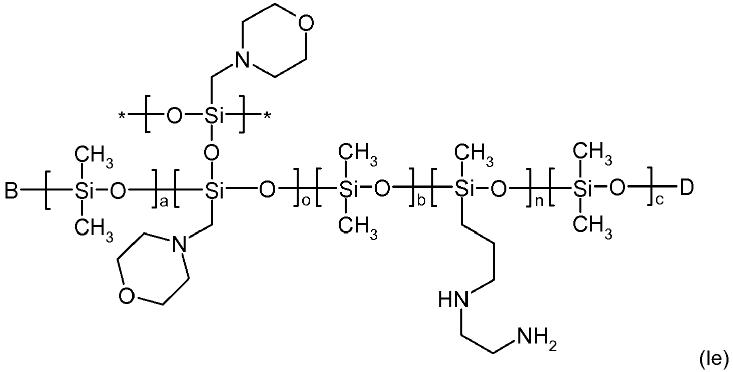

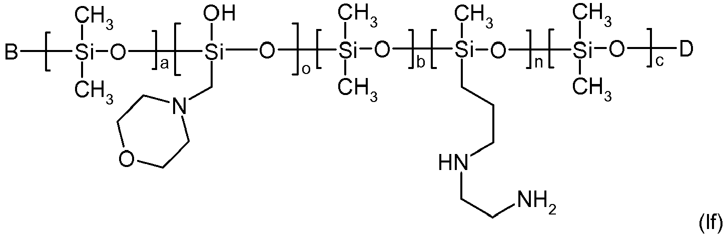

In der Struktureinheit (III) kann der Rest A
- für eine über ein -O- gebundene Struktureinheit (I), (II) oder (III) oder
- einen über ein -O- gebundenen oligomeren oder polymeren Rest enthaltend Struktureinheiten der Formeln (I), (II) oder (III)
- oder die Hälfte eines verbindenden O-Atoms zu einer Struktureinheit (III) oder für -OH stehen.

Im erstgenannten Fall wird die Struktureinheit (III) zu einer der Struktureinheiten (IIIa), (IIIb) oder (IIIc): 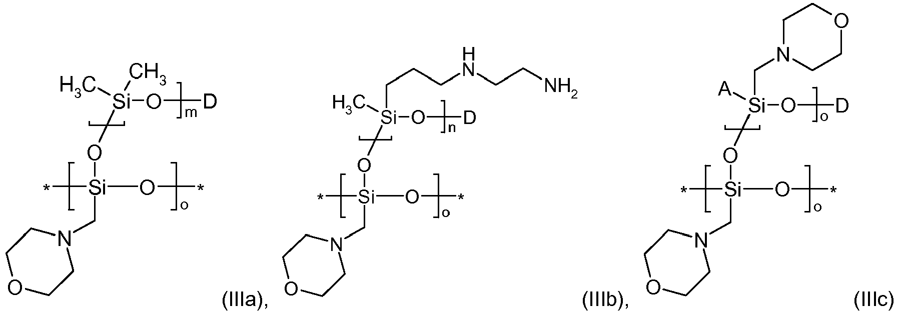 mit m = n = o = 1 und A bzw. D wie vorstehend definiert.

Im zweiten Fall können in den vorstehend genannten Formeln (IIIa), (IIIb) und (IIIc) die Indices m, n und o für ganze Zahlen zwischen 2 und 1000 stehen. Der zweite Fall deckt aber auch oligomere oder polymere Reste, ab, die mindestens zwei verschiedene Struktureinheiten der Formeln (I), (II) oder (III) enthalten, wie in Formel (IIId) dargestellt: 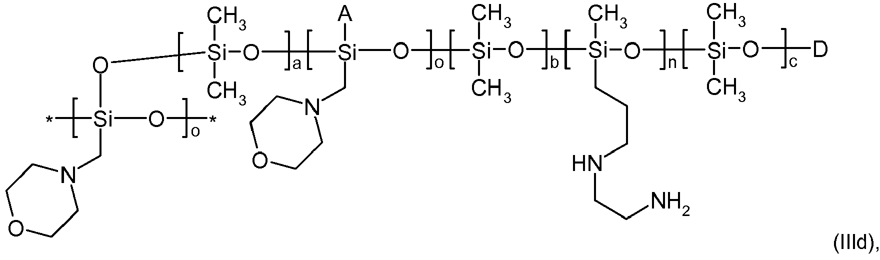 in der a, b und c für ganze Zahlen zwischen 0 und 1000 stehen, mit der Maßgabe a + b + c > 0 sowie n und o für ganze Zahlen zwischen 1 und 1000 stehen.

Im dritten Fall steht A die Hälfte eines verbindenden O-Atoms zu einer Struktureinheit (III) (dargestellt in der Struktureinheit (III) oder für -OH (dargestellt in der Struktureinheit (III)

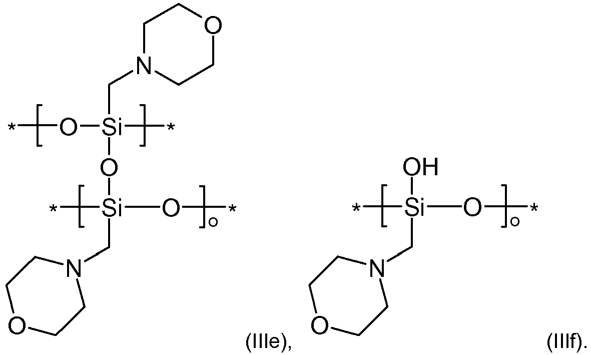

Die Struktureinheit (III) bzw. die Siloxaneinheiten o in der Formel (I) können über die Gruppe A Nest- bzw. Teilkäfigstrukturen ausbilden, wenn A für die Hälfte eines verbindenden O-Atoms zu einer Struktureinheit (III) steht. Erfindungsgemäße Haarbehandlungsmittel, die Silikone mit entsprechenden 4-morpholinomethyl-substituierten Silsequioxan-Teilstrukturen beinhalten, sind erfindungsgemäß bevorzugt, da diese Silikone zu enorm verbesserten Kämmbarkeiten und drastisch reduzierten Kontaktwinkeln führen.

Erfindungsgemäß bevorzugte Mittel sind demnach dadurch gekennzeichnet, dass die Zusammensetzung (A) mindestens ein 4-morpholinomethyl-substituiertes Silikon enthält, welches Struktureinheiten der Formel (II) aufweist 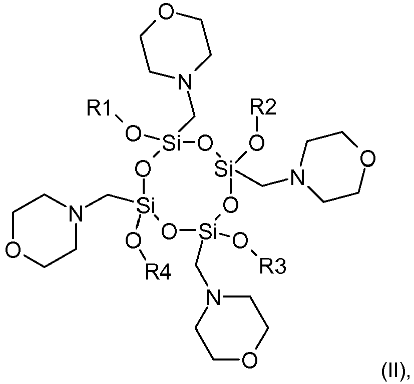 in der
R1, R2, R3 und R4 unabhängig voneinander für -H, -CH₃, eine Gruppe D, eine Struktureinheit (I), (II) oder (III) oder einen oligomeren oder polymeren Rest enthaltend Struktureinheiten der Formeln (I), (II) oder (III) stehen oder
zwei der Reste R1, R2, R3 und R4 für eine Struktureinheit -Si(R6)(R5)- mit
   R5 = -CH₃ oder eine Struktureinheit der Formel (I) oder (II) oder (III) oder einen oligomeren oder polymeren Rest enthaltend Struktureinheiten der Formeln (I), (II) oder (III)
   R6 = -OH, -CH₃, oder eine Struktureinheit der Formel (I) oder (II) oder (III) oder einen oligomeren oder polymeren Rest enthaltend Struktureinheiten der Formeln (I), (II) oder (III).

In bevorzugten Silikonen der Formel (II) steht mindestens einer der Reste R1, R2, R3 oder R4 für einen oligomeren oder polymeren Rest enthaltend Struktureinheiten der Formeln (I), (II) oder (III).

In weiter bevorzugten Silikonen der Formel (II) steht mindestens einer der Reste R1, R2, R3 oder R4 für einen oligomeren oder polymeren Rest enthaltend Struktureinheiten der Formeln (I) und (II).

In noch weiter bevorzugten Silikonen der Formel (II) steht mindestens einer der Reste R1, R2, R3 oder R4 für einen oligomeren oder polymeren Rest enthaltend Struktureinheiten der Formeln (I) und (II)und (III).

Vorzugsweise steht mindestens einer der Reste R1, R2, R3 oder R4 für eine -[-Si(CH₃)₂-O]ₘ-Gruppierung, d.h. ein Oligo- bzw. Polymeres der Struktureinheit (I). Vorzugsweise ist darüber hinaus die Struktureinheit (II) bzw. ein Oligo- oder Polymeres davon nie allein, sondern immer in statistischer Verteilung mit weiteren Struktureinheiten der Formel (I) als einer der Reste R1, R2, R3 oder R4 im Molekül gebunden.

Unabhängig davon, welches spezielle 4-Morpholinomethyl-substituierte Silikon in der Zubereitung (A) eingesetzt wird, sind erfindungsgemäße Mittel bevorzugt, die in der Zubereitung (A) ein 4-Morpholinomethyl-substituiertes Silikon enthalten, in dem mehr als 50 Mol-% der Struktureinheiten Dimethylsiloxy-Einheiten sind, d.h. in denen die Struktureinheit (I) mindestens die Hälfte aller Struktureinheiten des eingesetzten Silikons ausmacht.

In anderen Worten sind Silikone bevorzugt, bei denen m > (n + o) bzw. (a+b+c) > (n + o) gilt.

Noch weiter bevorzugte Mittel enthalten in der Zubereitung (A) ein 4-Morpholinomethyl-substituiertes Silikon, in dem mehr als 90 Mol-% der Struktureinheiten Dimethylsiloxy-Einheiten sind, d.h. in denen die Struktureinheit (I) mindestens Neun zehntel aller Struktureinheiten des eingesetzten Silikons ausmacht.

In anderen Worten sind Silikone bevorzugt, bei denen m > 10(n + o) bzw. (a+b+c) > 10(n + o) gilt. Noch weiter bevorzugte Kosmetische Mittel enthalten ein 4-Morpholinomethyl-substituiertes Silikon, in dem mehr als 98 Mol-% der Struktureinheiten Dimethylsiloxy-Einheiten sind, d.h. in denen die Struktureinheit (I) mindestens Achtundneunzig Hundertstel aller Struktureinheiten des eingesetzten Silikons ausmacht.

In anderen Worten sind Silikone bevorzugt, bei denen m > 50(n + o) bzw. (a+b+c) > 50(n + o) gilt. Noch weiter bevorzugte Kosmetische Mittel enthalten ein 4-Morpholinomethyl-substituiertes Silikon, in dem mehr als 98,5 Mol-% der Struktureinheiten Dimethylsiloxy-Einheiten sind, d.h. in denen die Struktureinheit (I) mindestens Neunhundertfünfundachtzig Tausendst aller Struktureinheiten des eingesetzten Silikons ausmacht.

In anderen Worten sind Silikone bevorzugt, bei denen m > 75(n + o) bzw. (a+b+c) > 75(n + o) gilt. Noch weiter bevorzugte Kosmetische Mittel enthalten ein 4-Morpholinomethyl-substituiertes Silikon, in dem mehr als 99 Mol-% der Struktureinheiten Dimethylsiloxy-Einheiten sind, d.h. in denen die Struktureinheit (I) mindestens Neun zehntel aller Struktureinheiten des eingesetzten Silikons ausmacht.

In anderen Worten sind Silikone bevorzugt, bei denen m > 100(n + o) bzw. (a+b+c) > 100(n + o) gilt. Zusammenfassend sind bevorzugte erfindungsgemäße Mittel dadurch gekennzeichnet, dass sie in der Zubereitung (A) mindestens ein 4-morpholinomethyl-substituiertes Silikon enthalten, bei dem
- m > (n + o) bzw. (a+b+c) > (n + o), vorzugsweise
- m > 10(n + o) bzw. (a+b+c) > 10(n + o), besonders bevorzugt
- m > 50(n + o) bzw. (a+b+c) > 50(n + o), weiter bevorzugt
- m > 75(n + o) bzw. (a+b+c) > 75(n + o) und insbesondere
- m > 100(n + o) bzw. (a+b+c) > 100(n + o) gilt.

Es hat sich gezeigt, dass die Wirkung der erfindungsgemäß eingesetzten Silikone noch gesteigert werden kann, wenn bestimmte nichtionische Komponenten ebenfalls in den erfindungsgemäßen Mitteln eingesetzt werden. Zudem haben diese nichtionischen Komponenten positive Effekte auf die Lagerstabilität der erfindungsgemäßen Mittel. Nichtionische Komponenten, die hier besonders geeignet sind, sind Ethoxylate von Decanol, Undecanol, Dodecanol, Tridecanol usw.. Als besonders geeignet haben sich ethoxylierte Tridecanole erwiesen, die mit besonderem Vorzug in die erfindungsgemäßen Mittel inkorporiert werden. Erfindungsgemäß besonders bevorzugte kosmetische Zusammensetzungen enthalten - bezogen auf ihr Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% verzweigtes, ethoxyliertes Tridecanol (INCI-Bezeichnung: Trideceth-5) oder α-iso-Tridecyl-ω-hydroxypolyglycolether (INCI-Bezeichnung: Trideceth-10) oder deren Mischungen.

Erfindungsgemäß bevorzugte morpholinomethyl-substituierte(s) Silikon(e) weisen sowohl Hydroxy- als auch Alkoxygruppen auf. Erfindungsgemäß besonders bevorzugte Mittel enthalten in der Zubereitung (A) hydroxy-terminierte(s) 4-morpholinomethyl-substituierte(s) Silikon(e), in denen das Molverhältnis Hydroxy/Alkoxy im Bereich von 0,2:1 bis 0,4:1, vorzugsweise im Bereich von 1:0,8 bis 1:1,1 liegt. Die mittleren Molekulargewicht des Silikons beträgt vorzugsweise von 2.000 bis 200.000 und noch mehr bevorzugt von 5.000 bis 100.000, insbesondere 10.000 bis 50.000 Dalton. Kosmetische Zusammensetzungen, bei denen die gewichtmittlere Molmasse des in ihnen enthaltenen 4-morpholinomethyl-substituierten Silikons der Formel (I) im Bereich von 2.000 bis 1.000.000 gmol⁻¹, vorzugsweise im Bereich von 5.000 bis 200.000 gmol⁻¹ liegt, sind bevorzugt.

Die mittleren Molekulargewichte von aminosubstituierten Silikonen sind beispielsweise durch Gelpermeationschromatographie (GPC) bei Raumtemperatur in Polystyrol messbar. Als Säulen können Styragel Spalten µ, als Eluent THF und als Flussrate 1 ml / min gewählt werden. Die Detektion erfolgt vorzugsweise mittels Refraktometrie und UV-Meter.

Erfindungsgemäß enthalten die Zubereitung (B) und/oder Zubereitung (C) weiterhin mindestens ein natürliches Polymer (siehe unten). Zubereitung (A) ist vorzugsweise frei von Xanthan, da es sich gezeigt hat, dass dies die Aufhellungsleistung verbessert.

Erfindungsgemäß bevorzugte Mittel sind dadurch gekennzeichnet, dass Zubereitung (A) frei von Xanthan ist und - bezogen auf ihr Gewicht - 5 bis 60 Gew.-%, vorzugsweise 10 bis 55 Gew.-%, besonders bevorzugt 15 bis 50 Gew.-% und insbesondere 20 bis 45 Gew.-% mindestens eines Persulfat, ausgewählt aus Ammoniumperoxodisulfat und/oder Kaliumperoxodisulfat und/oder Natriumperoxodisulfat, enthält.

Der erste Gegenstand der Erfindung umfasst Mittel zum Aufhellen keratinischer Fasern, die mindestens zwei getrennt voneinander verpackte Zubereitungen (A) und (B) sowie gegebenenfalls eine weitere getrennt von (A) und (B) verpackte Zubereitung (C) enthalten. Die Zubereitungen (B) und gegebenenfalls (C) enthalten die Wirkstoffe in einem fließfähigen, kosmetischen Träger. Die Grundlage des fließfähigen, kosmetischen Trägers ist dabei bevorzugt wässrig oder wässrig-alkoholisch. Zum Zwecke der Haarbleiche sind solche Träger beispielsweise transparente Gele oder auch tensidhaltige schäumende Lösungen, wie beispielsweise Shampoos, Schaumaerosole oder andere Zubereitungen, die für die Anwendung auf dem Haar geeignet sind. Ein bevorzugter, fließfähiger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether enthalten. Bevorzugt sind dabei alle wasserlöslichen organischen Lösungsmittel.

Als wesentlichen Inhaltstoff enthalten die Zubereitungen (B) und/oder die Zubereitungen (C) des Blondiermittels gemäß der Erfindung mindestens ein Oxidationsmittel.

In einer besonderen Ausführungsform enthalten die Zubereitungen (B) gemäß der Erfindung Wasserstoffperoxid als Oxidationsmittel.

Die Konzentration einer Wasserstoffperoxid-Lösung in der Oxidationsmittelzubereitung (B) wird einerseits von den gesetzlichen Vorgaben und andererseits von dem gewünschten Effekt bestimmt.

Vorzugsweise enthalten die Zubereitungen (B) bezogen auf ihr Gewicht Wasserstoffperoxid in Mengen von 0,5 bis 30 Gew.-%, bevorzugt von 1 bis 20 Gew.-%, besonders bevorzugt von 5 bis 15 Gew.-% und insbesondere bevorzugt von 6 bis 12 Gew.-%, explizit 6, 7, 8, 9, 10, 11 oder 12 Gew.-%.

Erfindungsgemäß bevorzugte anwendungsbereite Mittel sind dadurch gekennzeichnet, dass sie, bezogen auf das Gesamtgewicht des anwendungsbereiten Mittels, 0,01 bis 12 Gew.-%, vorzugsweise 0,1 bis 10 Gew.-%, besonders bevorzugt 1 bis 8 Gew.-% Wasserstoffperoxid enthalten.

Erfindungsgemäß bevorzugte anwendungsbereite Mittel sind dadurch gekennzeichnet, dass sie, bezogen auf das Gesamtgewicht des anwendungsbereiten Mittels, 0,01 bis 12 Gew.-%, vorzugsweise 0,1 bis 10 Gew.-%, besonders bevorzugt 1 bis 8 Gew.-% Wasserstoffperoxid enthalten.

Zur Stabilisierung des Wasserstoffperoxid kann der pH-Wert der Zubereitung (B) bevorzugt auf pH 3 bis 5, besonders bevorzugt auf pH 3,5 bis 4,5 und insbesondere bevorzugt auf pH 3,8 bis 4,2 eingestellt werden.

Zubereitung (B) (und/oder Zubereitung (C)) können darüber hinaus zur Viskositästregulierung ein natürliches Polymer enthalten. Wenn das Mittel zum Aufhellen keratinischer Fasern, genau zwei getrennt voneinander verpackte Zubereitungen (A) und (B) enthält, die unmittelbar vor der Anwendung zu einer Anwendungsmischung vermischt werden, so enthält Zubereitung (B) gemäß dieser Ausführungsform der Erfindung mindestens ein natürliches Polymer.

Wenn das Mittel zum Aufhellen keratinischer Fasern, mindestens drei getrennt voneinander verpackte Zubereitungen (A), (B) und (C) enthält, die unmittelbar vor der Anwendung zu einer Anwendungsmischung vermischt werden, so können Zubereitung (B) und/oder Zubereitung (C) mindestens ein natürliches Polymer enthalten.

Als natürliches Polymer können beispielsweise Cellulosederivate verwendet werden, die als Verdickungsmittel Verwendung finden. Beispiele sind Agar-Agar, Carrageen, Alginate, Xanthan-Gum, Karaya-Gummi, Ghatti-Gummi, Tragant, Skleroglucangums oder Gummi arabicum, Alginate, Pektine, Polyosen, Guar-Gums, Johannisbrotbaumkernmehl, Leinsamengummen, Dextrane, Pektine, Staerke-Fraktionen und Derivate wie Amylose, Amylopektin und Dextrine, Gelatine und Casein sowie Cellulosederivate, wie beispielsweise Methylcellulose, Carboxyalkylcellulosen wie Carboxymethylcellulose und Hydroxyalkylcellulosen wie Hydroxyethylcellulose.

Natürliche Polymere aus den genannten Substanzklassen sind kommerziell erhältlich und werden beispielsweise unter den Handelsnamen Deuteron®-XG (anionisches Heteropolysaccharid auf Basis von β-D-Glucose, D-Manose, D-Glucuronsaeure, Schoener GmbH), Deuteron®-XN (nichtionogenes Polysaccharid, Schoener GmbH), Protanal RF 6650 alginate (Natriumalginat, FMC Biopolymer), Cekol (Cellulose Gum, Kelco), Kelzan (Xanthan-Biopolymer, Kelco), Xanthan FN (Xanthan-Biopolymer, Jungbunzlauer), Keltrol z.B. Keltrol CG-T (Xanthan-Biopolymer, Kelco) oder Keltrol CG-SFT (Xanthan-Biopolymer, Kelco) angeboten.

In einer bevorzugten Ausführungsform der Erfindung enthalten die Zubereitungen (B) und/oder gegebenenfalls (C) Xanthan.

Erfindungsgemäß bevorzugt sind solche Xanthane, die nach der Quellung transparente Zubereitungen ergeben. Insbesondere bevorzugt ist die Verwendung des Xanthan-Biopolymers, welches unter dem Handelsnamen Keltrol CG-SFT der Firma Kelco vertrieben wird.

In einer bevorzugten Ausführungsform enthält Zubereitung (B) bezogen auf ihr Gewicht Xanthan in Mengen von 0,1 bis 10 Gew.-%, vorzugsweise von 0,5 bis 6 Gew.-%, besonders bevorzugt von 0,7 bis 5 Gew.-% und insbesondere bevorzugt von 1 bis 4 Gew.-%, explizit 1, 2, 3 oder 4 Gew.-%, wenn das Mittel zum Aufhellen keratinischer Fasern, genau zwei getrennt voneinander verpackte Zubereitungen (A) und (B) enthält, die unmittelbar vor der Anwendung zu einer Anwendungsmischung vermischt werden. Vorzugsweise enthalten die fertigen vermischten Anwendungszubereitungen bezogen auf ihr Gewicht, Xanthan in Mengen von 0,6 bis 5 Gew.-%, besonders bevorzugt von 1,0 bis 3,5 Gew.-% und insbesondere bevorzugt von 1,5 bis 2,5 Gew.-% explizit 1,5; 1,6; 1,7; 1,8; 1,9; 2,0; 2,1; 2,2; 2,3; 2,4 oder 2,5 Gew.-%.

Wenn das Mittel zum Aufhellen keratinischer Fasern, mindestens drei getrennt voneinander verpackte Zubereitungen (A), (B) und (C) enthält, die unmittelbar vor der Anwendung zu einer Anwendungsmischung vermischt werden, so können Zubereitung (B) und/oder Zubereitung (C) Xanthan enthalten. Wenn das Mittel zum Aufhellen keratinischer Fasern, mindestens drei getrennt voneinander verpackte Zubereitungen (A), (B) und (C) enthält, die unmittelbar vor der Anwendung zu einer Anwendungsmischung vermischt werden, sind Zubereitungen bevorzugt, in denen Zubereitung (C) Xanthan enthält. Insbesondere bevorzugt sind Zubereitungen, in denen Zubereitung (C) Xanthan enthält und Zubereitung (B) frei von Xanthan ist.

Unabhängig davon, ob Zubereitung (B) und/oder Zubereitung (C) Xanthan enthalten, sind solche fertig vermischten Anwendungszubereitungen bevorzugt, die, bezogen auf das Gewicht der fertigen Anwendungszubereitung, Xanthan in Mengen von 0,6 bis 5 Gew.-%, besonders bevorzugt von 1,0 bis 3,5 Gew.-% und insbesondere bevorzugt 1,5 bis 2,5 Gew.-% explizit 1,5; 1,6; 1,7; 1,8; 1,9; 2,0; 2,1; 2,2; 2,3; 2,4 oder 2,5 Gew.-%.

Erfindungsgemäß besonders bevorzugte Mittel sind dadurch gekennzeichnet, dass die Zubereitung (B) - bezogen auf ihr Gewicht - 0,1 bis 10 Gew.-%, vorzugsweise 0,25 bis 7,5 Gew.-%, weiter bevorzugt 0,5 bis 5 Gew.-%, besonders bevorzugt 0,75 bis 4 Gew.-% und insbesondere 1 bis 2,5 Gew.-% Xanthan und 0,5 bis 30 Gew.-%, vorzugsweise 1 bis 20 Gew.-%, besonders bevorzugt 5 bis 15 Gew.-% und insbesondere 6 bis 12 Gew.-% Wasserstoffperoxid, berechnet als 100%iges H₂O₂, enthält.

Die viskosen Eigenschaften von Zubereitung (B) sind von Bedeutung für ihre gute Mischbarkeit und hohe Stabilität. In einer bevorzugten Ausführungsform sind die Zubereitungen (B) der vorliegenden Erfindung dadurch gekennzeichnet, dass sie eine Viskosität von 1.000 mPa·s bis 50.000 mPa·s, vorzugsweise von von 5.000 mPa·s bis 45.000 mPa·s und besonders bevorzugt von 7.000 mPa·s bis 40.000 mPa·s bei Messungen mit einem Rotationsviskosimeter von Brookfield, Spindelgröße 4, bei 25°C und 4 Upm, aufweisen. Die fertig angemischten und anwendungsbereiten Mittel weisen vorzugsweise eine Viskosität von 10.000 mPa·s bis 50.000 mPa·s und besonders bevorzugt von 18.000 mPa·s bis 30.000 mPa·s bei Messungen mit einem Rotationsviskosimeter von Brookfield, Spindelgröße 5, bei 25°C und 4 Upm auf.

Weiterhin ist die Einstellung des pH-Wertes von Bedeutung für gute Mischbarkeit und Stabilität. Erfindungsgemäß bevorzugt sind fertig angemischte und anwendungsbereite Mittel, deren pH-Wert zwischen 9 und 12 liegt.

Weiterhin kann es erfindungsgemäß von Vorteil sein, wenn Zubereitung (B) mindestens ein nichtionisches Tensid, bevorzugt mindestens einen ethoxylierten Fettalkohol mit 40 bis 60 Ethylenoxideinheiten enthält. Erfindungsgemäß ist darunter ein Anlagerungsprodukt von Ethylenoxid an einen Fettalkohol zu verstehen. Fettalkohole sind dabei gesättigte und ungesättigte Alkohole mit 12 bis 24 C-Atomen, welche linear oder verzweigt sein können. Die Molmenge an Ethylenoxid, die pro Mol Fettalkohol eingesetzt wurde, bezeichnet dabei den Ethoxylierungsgrad verstanden. Als nichtionisches Tensid eignen sich dabei insbesondere Ethylenoxid-Anlagerungsprodukte an Octylalkohol (Caprylalkohol), Nonylalkohol (Pelargonylalkohol), Undecylalkohol, Undec-10-en-1-ol, Dodecylalkohol (Laurylalkohol), 2,6,8-Trimethyl-4-nonanol (Isolaurylalkohol), Tridecylalkohol, Tetradecylalkohol (Myristylalkohol), Pentadecylalkohol, Hexadecylalkohol (Cetyl-/ Palmitylalkohol), Heptadecylalkohol, Octadecylalkohol (Stearylalkohol), Isostearylalkohol, (9*Z*)-Octadec-9-en-1-ol (Oleylalkohol), (9*E*)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenylalkohol), Nonadecan-1-ol (Nonadecylalkohol), Eicosan-1-ol (Eicosylalkohol /Arachylalkohol), (9*Z*)-Eicos-9-en-1-ol (Gadoleylalkohol), (5*Z*,8*Z*,11*Z*,14*Z*)-Eicosa-5,8,11,14-tetraen-1-ol (Arachidonalkohol), Heneicosylalkohol, Docosylalkohol (Behenylalkohol), (13*Z*)-Docos-13-en-1-ol (Erucylalkohol) oder (13*E*)-Docosen-1-ol (Brassidylalkohol). Es ist erfindungsgemäß ebenfalls möglich, Gemische von Fettalkoholen, die durch gezielte Mischung oder auch durch Gewinnungsverfahren als solche anfallen, einzusetzen. Beispiele sind Cocosalkohol (Mischung aus C₈-C₁₈-Fettalkoholen) oder Cetearylalkohol (1:1-Mischung aus C₁₆- und C₁₈-Fettalkoholen).

Bevorzugt sind Ethoxylierungsgrade von 20 bis 60. Erfindungsgemäß bevorzugte nichtionische Tenside vom Typ ethoxylierter Fettalkohol sind Ceteareth-20 und Ceteareth-50.

Weiterhin können die Blondiermittel Alkalisierungsmittel enthalten. Bevorzugte Alkalisierungsmittel sind beispielsweise Ammoniak, Alkanolamine, basischen Aminosäuren, sowie anorganischen Alkalisierungsmittel wie (Erd-)Alkalimetallhydroxide, (Erd-)Alkalimetallmetasilikate, (Erd-) Alkalimetallphosphate und (Erd-)Alkalimetallhydrogenphosphate. Als Metallionen dienen bevorzugt Lithium, Natrium und/oder Kalium. Insbesondere bevorzugtes Alkalisierungsmittel ist Ammoniak.

Erfindungsgemäß einsetzbare, anorganische Alkalisierungsmittel sind bevorzugt ausgewählt aus Natriumhydroxid, Kaliumhydroxid, Calciumhydroxid, Bariumhydroxid, Natriumphosphat, Kaliumphosphat, Natriumsilicat, Kaliumsilicat, Magnesiumsilicat, Natriumcarbonat und Kaliumcarbonat. Besonders bevorzugt sind Natriumhydroxid und/oder Kaliumhydroxid.

Erfindungsgemäß einsetzbare Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Methylglucamin, Triethanolamin, Diethanolamin und Triisopropanolamin. Insbesondere bevorzugte Alkanolamine sind Monoethanolamin, 2-Amino-2-methyl-propanol und Triethanolamin.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin, L-Ornithin, D- Ornithin, D/L- Ornithin, L-Histidin, D-Histidin und/oder D/L-Histidin.

Besonders bevorzugt werden L-Arginin, D-Arginin und/oder D/L-Arginin als ein Alkalisierungsmittel im Sinne der Erfindung eingesetzt.

Manche Kunden empfinden die intensive Geruchsbildung von Ammoniak belästigend oder störend. Obwohl Ammoniak ein bevorzugtes Alkalisierungsmittel ist, können daher anwendungsbereite Zubereitungen erfindungsgemäß bevorzugt sein, die frei von Ammoniak sind. Bevorzugte Alkalisierungsmittel für Zubereitungen, die frei von Ammoniak sind, sind Monoethanolamin, 2-Amino-2-methyl-propanol und Triethanolamin.

Wenn die anwendungsbereiten Mischungen Alkalisierungsmittel enthalten, sind Zubereitungen erfindungsgemäß bevorzugt, die Alkalisierungsmittel in einer Menge von 0,05 bis 20 Gew.-%, insbesondere von 0,5 bis 10 Gew.-%, jeweils bezogen auf das Gesamtgewicht des anwendungsbereiten Mittels, enthalten.

In einer weiteren Ausführungsform der Erfindung, können Zubereitungen (A) und (B) mit weiteren separat verpackten Zubereitungen unmittelbar vor der Anwendung zu einer Anwendungsmischung vermischt werden.

In einer bevorzugten Ausführungsform der Erfindung enthält das erfindungsgemäße Mittel zusätzlich mindestens eine weitere getrennt von Zubereitungen (A) und (B) verpackte Zubereitung (C), wobei Zubereitung (C) mindestens ein Alkalisierungsmittel und mindestens ein natürliches Polymer enthält.

Bevorzugt enthält Zubereitung (C) natürliche Polymere, die bereits weiter oben im Text im Zusammenhang mit Zubereitung (B) genannt wurden.

Erfindungsgemäß bevorzugt sind solche Alkalisierungsmittel, die bereits zuvor beschrieben wurden. Wenn die Zubereitungen (C) Alkalisierungsmittel enthalten, sind Zubereitungen erfindungsgemäß bevorzugt, die Alkalisierungsmittel in einer Menge von 0,05 bis 20 Gew.-%, insbesondere von 0,5 bis 10 Gew.-%, jeweils bezogen auf das Gesamtgewicht des anwendungsbereiten Mittels, enthalten.

Unabhängig davon, ob Zubereitungen (C) und/oder Zubereitung (B) und/oder weitere Zubereitungen Alkalisierungsmittel enthalten, sind, wenn Alkalisierungsmittel zum Einsatz kommen, solche Zubereitungen erfindungsgemäß bevorzugt, die Alkalisierungsmittel in einer Menge von 0,05 bis 20 Gew.-%, insbesondere von 0,5 bis 10 Gew.-%, jeweils bezogen auf das Gesamtgewicht des anwendungsbereiten Mittels, enthalten.

Zur weiteren Steigerung der Aufhellleistung kann Zubereitung (C) zusätzlich als Bleichverstärker eine Silicium-haltige Verbindung zugesetzt werden. Diese wird bevorzugt ausgewählt aus der Gruppe, die gebildet wird aus Kieselsäure, Alkalimetallsilicaten und Erdalkalimetallsilicaten.

Obwohl bereits geringe Mengen der Silicium-haltigen Verbindungen die Aufhellleistung erhöhen, kann es erfindungsgemäß bevorzugt sein, die Silicium-haltigen Verbindungen in Mengen von 0,05 Gew.-% bis 50 Gew.-%, bevorzugt in Mengen von 0,5 Gew.-% bis 30 Gew.-% und besonders bevorzugt in Mengen von 1,0 Gew.-% bis 25 Gew.-%, jeweils bezogen auf das Gesamtgewicht der Zubereitung (C), einzusetzen.

Als Silicium-haltige Verbindungen werden insbesondere Alkalimetallsilicate in Form von Wasserglas eingesetzt. Unter Wasserglas ist dabei eine Verbindung zu verstehen, die aus einem Silicat der Formel (SiO₂)ₙ(Na₂O)ₘ(K₂O)ₚ gebildet wird, wobei n für eine positive rationale Zahl und m und p unabhängig voneinander für eine positive rationale Zahl oder für 0 stehen, mit den Maßgaben, dass mindestens einer der Parameter m oder p von 0 verschieden ist und das Verhältnis zwischen n und der Summe aus m und p zwischen 1:1 und 4:1 liegt.

Neben den durch die Summenformel beschriebenen Komponenten können die Wassergläser in geringen Mengen noch weitere Zusatzstoffe, wie Phosphate oder Magnesiumsalze, enthalten.

Erfindungsgemäß besonders bevorzugte Wassergläser werden unter anderem unter den Bezeichnungen Ferrosil^{®} 119, Natronwasserglas 40/42, Portil^{®} A, Portil^{®} AW und Portil^{®} W und Britesil^{®} C20 vertrieben.

Weiterhin können als Silicium-haltige Verbindungen insbesondere Kieselsäuren eingesetzt werden, die auch als Silica oder Kieselgel vertrieben werden. Bevorzugt ist dabei ein Kieselgel, welches unter dem Handelsnamen Aerosil 200 (INCI-Bezeichnung: Silica) vertrieben wird.

Weiterhin können die Aufhell- bzw. Blondiermittel zur Verstärkung der Blondierwirkung noch zusätzliche Bleichkraftverstärker enthalten. Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. i-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Als Bleichverstärker vom Typ der Kohlensäurederivate können erfindungsgemäß bevorzugt Carbonatsalze bzw. Hydrogencarbonatsalze eingesetzt werden. Diese sind bevorzugt ausgewählt aus der Gruppe der Ammonium-, Alkalimetall- (insbesondere Na und K) sowie Erdalkalimetall- (insbesondere Mg und Ca), -carbonatsalze bzw. -hydrogencarbonatsalze. Besonders bevorzugte Carbonat- bzw. Hydrogencarbonatsalze sind Ammoniumhydrogencarbonat, Ammoniumcarbonat, Natriumhydrogencarbonat, Natriumcarbonat, Kaliumhydrogencarbonat, Kaliumcarbonat, Magnesiumcarbonat und Calciumcarbonat. Diese besonders bevorzugten Salze können allein oder in deren Mischungen von mindestens zwei Vertretern als Bleichverstärker verwendet werden. Weiterhin sind Alkylcarbonate, -carbamate, Silylcarbonate sowie -carbamate geeignete Bleichverstärker.

Weiterhin erfindungsgemäß einsetzbare Bleichkraftverstärker können aus stickstoffhaltigen, gegebenenfalls kationischen Heterocyclen, insbesondere Imidazol, ausgewählt werden.

Besonders bevorzugte stickstoffhaltige, heterocyclische Bleichkraftverstärker sind die quaternierten Kationen von Pyridinen und 3,4-Dihydroisochinolinen, wie Salze des 4-Acetyl-1-methylpyridiniums, insbesondere 4-Acetyl-1-methylpyridinium-p-toluolsulfonat, Salze des 2-Acetyl-1-methylpyridiniums, insbesondere 2-Acetyl-1-methylpyridinium-p-toluolsulfonat, sowie Salze des N-Methyl-3,4-dihydroisochinoliniums, insbesondere N-Methyl-3,4-dihydroisochinolinium-p-toluolsulfonat.

Weiterhin erfindungsgemäß einsetzbarer Bleichkraftverstärker ist Harnstoff.

Bleichkraftverstärker können in Zubereitung (A) und/oder Zubereitung (B) und/oder gegebenenfalls Zubereitung (C) und/oder gegebenenfalls weiteren Zubereitungen enthalten sein. Dabei können die Bleichkraftverstärker entweder in nur einer der Zubereitungen oder in zwei oder mehr der Zubereitungen enthalten sein. Hydrolyseempfindliche Bleichkraftverstärker können bevorzugt in der pulverförmigen Zubereitung (A) eingesetzt werden. Unabhängig davon, ob Bleichkraftverstärker in Zubereitung (A) und/oder Zubereitung (B) und/oder Zubereitung (C) und/oder weiteren Zubereitungen eingesetzt werden, sind diese, wenn Bleichkraftverstärker zum Einsatz kommen, bevorzugt in Mengen von 0,5 bis 30 Gew.-%, insbesondere in Mengen von 2 bis 20 Gew.-%, jeweils bezogen auf das Gesamtgewicht der fertig angemischten Blondierzubereitung, enthalten.

Weiterhin können die Aufhell- bzw. Blondiermittel zur Mattierung von unerwünschten Restfarbeindrücken, insbesondere im rötlichen oder bläulichen Bereich, bestimmte, direktziehende Farbstoffe der komplementären Farben enthalten. Dabei handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Direktziehende Farbstoffe sind als anionische, kationische und nichtionische direktziehende Farbstoffe bekannt. Die direktziehenden Farbstoffe werden jeweils bevorzugt in einer Menge von 0,001 bis 2 Gew.-%, bezogen auf die gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe. Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Ganz besonders bevorzugt sind Mittel, die mindestens eine Kombination aus Tetrabromphenolblau und Acid Red 92 enthalten.

Weiterhin hat es sich als vorteilhaft erweisen, wenn die anwendungsbereiten Mittel mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Diese können unterschiedlichen chemischen Gruppen angehören. Vorzugsweise werden einzeln oder im Gemisch miteinander eingesetzt. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide, insbesondere Arginin und/oder Serin; Proteinhydrolysate auf tierischer und/oder pflanzlicher Basis, wie beispielsweise Elastin-, Kollagen-, Keratin-, Seiden- und Milcheiweiß-Proteinhydrolysate, oder Mandel-, Reis-, Erbsen-, Kartoffel- und Weizenproteinhydrolysate, sowie in Form ihrer Fettsäure-Kondensationsprodukte oder gegebenenfalls anionisch oder kationisch modifizierten Derivate; Lichtschutzmittel, wie derivatisierte Benzophenone, Zimtsäure-Derivate und Triazine; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen, insbesondere der Gruppen A, B₃, B₅, B₆, C, E, F und H; Pflanzenextrakte; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft. Besonders bevorzugt gemäß der Erfindung sind solche Wirk-, Hilfs- und Zusatzstoffe, die in Kombination mit dem erfindungsgemäßen Mittel zu einer möglichst durchsscheinenden bis transparenten Anwendungsmischung führen.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

Ein zweiter Gegenstand der Erfindung ist ein Verfahren zur Farbveränderung keratinischer Fasern, bei dem mindestens zwei getrennt voneinander verpackte Zubereitungen (A) und (B), von welchen Zubereitung (A) mindestens ein Persulfat, mindestens ein Acrylatpolymer und Carboxymethylcellulose und Zubereitung (B) mindestens ein Oxidationsmittel enthält, zu einer Anwendungsmischung vermischt werden, diese auf die Fasern aufgetragen wird und nach einer Einwirkungszeit wieder abgespült wird, dadurch gekennzeichnet, dass
i. Zubereitung (A) - bezogen auf ihr Gewicht -
   a1) 0 bis 3 Gew.-% Keratinhydrolysat(e) und/oder
   a2) 0 bis 5 Gew.-% Silikonöl(e) enthält,
mit der Maßgabe, dass die Zubereitung (A) - bezogen auf ihr Gewicht - 0,1 bis 6 Gew.-% Verbindung(en) aus den Gruppen a1) und a2) enthält.

Die anwendungsbereiten Mittel werden unmittelbar vor der Anwendung auf dem Haar durch Mischen der zwei Zubereitungen (A) und (B) und gegebenenfalls einer dritten Zubereitung (C) und/oder weiteren Zubereitungen hergestellt. Bei anwendungsbereiten Mitteln, die aus mehr als zwei Zubereitungen zu einer fertigen Anwendungsmischung vermischt werden, kann es unerheblich sein, ob zunächst zwei Zubereitungen miteinander vermischt werden und anschließend die dritte Zubereitung zugegeben und untergemischt wird, oder ob alle Zubereitungen gemeinsam zusammengeführt und anschließend vermischt werden. Das Vermischen kann durch Verrühren in einer Schale oder einem Becher erfolgen oder durch Schütteln in einem verschließbaren Behälter.

Der Begriff "unmittelbar" ist dabei als Zeitraum von wenigen Sekunden bis eine Stunde, vorzugsweise bis 30 min, insbesondere bis 15 min zu verstehen.

Die erfindungsgemäßen Mittel werden in einem Verfahren zum Aufhellen von keratinischen Fasern, insbesondere menschlichen Haaren, angewendet, bei dem das Mittel auf die keratinhaltigen Fasern aufgebracht, bei einer Temperatur von Raumtemperatur bis 45 °C für eine Einwirkdauer von 10 bis 60 Minuten auf der Faser belassen und anschließend mit Wasser wieder ausgespült oder mit einem Shampoo ausgewaschen wird.

Bevorzugt beträgt die Einwirkzeit der anwendungsbereiten Aufhellmittel 10 bis 60 min, insbesondere 15 bis 50 min, besonders bevorzugt 20 bis 45 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie mit Hilfe eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist ebenfalls erfindungsgemäß. Bevorzugt liegt die Temperatur während der Einwirkzeit zwischen 20°C und 40°C, insbesondere zwischen 25°C und 38°C. Die Aufhellmittel ergeben bereits bei physiologisch verträglichen Temperaturen von unter 45°C gute Blondier- und Aufhellergebnisse.

Nach Ende der Einwirkzeit wird die verbleibende Aufhellzubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Leitungswasser erfolgen kann, wenn das Aufhellmittel einen stark tensidhaltigen Träger besitzt.

Ein bevorzugtes Verfahren ist dadurch gekennzeichnet, dass die Kontrolle des Aufhellgrades der keratinischen Faser während der Einwirkzeit ohne Entfernen der Anwendungsmischung von der Faser visuell erfolgt. Dazu wird ein anwendungsbereites, vorzugsweise durchscheinendes bzw. transparentes Mittel des ersten Erfindungsgegenstands auf menschliches Haar aufgetragen, und der Aufhellprozess während der Einwirkzeit ein oder mehrmals durch visuelle Kontrolle, ohne Abtragen des Mittels von der Faser, beurteilt. Somit ist eine vereinfachte und permanente Kontrolle des Entfärbevorgangs gewährleistet.

"Transparent" im Sinne der Erfindung sind Zubereitungen, die bei Auftragung einer gleichmäßigen Schicht dieser Zubereitungen von 1 bis 3 mm Dicke auf einen Untergrund klar sind und so durchblickt werden können, dass das menschliche Auge die Farbe des Untergrunds ohne Trübung erkennen und beurteilen kann. Transparenz kann vom Fachmann weiterhin durch technische Methoden gemessen werden. "Transparent" im Sinne der Erfindung sind daher auch Zubereitungen, die bei photometrischen Messungen mit einem Methrom Photometer 662 bei 25°C Transmissionen von mindestens 70%, insbesondere mindestens 80%, erreichen.

Erfindungsgemäß bevorzugte Verfahren sind dadurch gekennzeichnet, dass die Kontrolle des Aufhellgrades der keratinischen Faser während der Einwirkzeit ohne Entfernen der Anwendungsmischung von der Faser visuell erfolgt.

Die bevorzugten Ausführungsformen des ersten Erfindungsgegenstands gelten mutatis mutandis auch für den zweiten Erfindungsgegenstand.

## Patentansprüche

1. Mittel zum Aufhellen keratinischer Fasern, enthaltend mindestens zwei getrennt voneinander verpackte Zubereitungen (A) und (B) sowie gegebenenfalls eine weitere getrennt von (A) und (B) verpackte Zubereitung (C), die unmittelbar vor der Anwendung zu einer Anwendungsmischung vermischt werden, wobei
i. Zubereitung (A) mindestens ein Persulfat, mindestens ein Acrylatpolymer und Carboxymethylcellulose enthält und
ii. Zubereitung (B) fließfähig ist und mindestens ein Oxidationsmittel enthält,
**dadurch gekennzeichnet, dass** Zubereitung (A) - bezogen auf ihr Gewicht -
a1) 0 bis 3 Gew.-% Keratinhydrolysat(e) und/oder
a2) 0 bis 5 Gew.-% Silikonöl(e) enthält,
mit der Maßgabe, dass die Zubereitung (A) - bezogen auf ihr Gewicht - 0,1 bis 6 Gew.-% Verbindung(en) aus den Gruppen a1) und a2) enthält.

2. Mittel nach Anspruch 1, **dadurch gekennzeichnet, dass** die Zubereitung (A) 0,01 bis 1 Gew.-%, vorzugsweise 0,05 bis 0,75 Gew.-%, weiter bevorzugt 0,1 bis 0,5 Gew.-%, besonders bevorzugt 0,15 bis 0,4 Gew.-% und insbesondere 0,2 bis 0,3 Gew.-% Hydrolysat(e) mit Molmassen von 400 bis 1200 Dalton, gewonnen aus dem Kortex und/oder der Cuticula von keratinischen Fasern, enthält.

3. Mittel nach einem der Ansprüche 1 oder 2, **dadurch gekennzeichnet, dass** die Zubereitung (A) 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% Silikonöl(e) aus der Gruppe der Verbindungen mit der INCI-Bezeichnung Dimethicone enthält.

4. Mittel nach einem der Ansprüche 1 bis 3, **dadurch gekennzeichnet, dass** die Zubereitung (B) - bezogen auf ihr Gewicht - 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% kosmetische(s) Öl(e), vorzugsweise Silikonöl(e), enthält.

5. Mittel nach einem der Ansprüche 1 bis 4, **dadurch gekennzeichnet, dass** 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% Silikonöl(e) aus der Gruppe der Verbindungen mit der INCI-Bezeichnung Amodimethicone enthält, wobei Verbindungen mit den INCI-Bezeichnungen Silicone Quaternium-1, Silicone Quaternium-2, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, Silicone Quaternium-16, Silicone Quaternium-17, Silicone Quaternium-18, Silicone Quaternium-20, Silicone Quaternium-21 oder Silicone Quaternium-22 bevorzugt sind.

6. Mittel nach einem der Ansprüche 1 bis 5, **dadurch gekennzeichnet, dass** die Zubereitung (A) 0,01 bis 5 Gew.-%, vorzugsweise 0,05 bis 4 Gew.-%, weiter bevorzugt 0,1 bis 2,5 Gew.-%, besonders bevorzugt 0,25 bis 2 Gew.-% und insbesondere 0,5 bis 1,5 Gew.-% Silikonöl(e) der Formel (I) enthält, 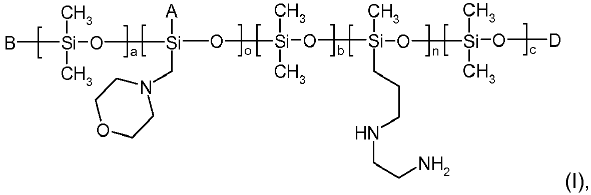 in der
A für eine über ein -O- gebundene Struktureinheit (I), (II) oder (III) 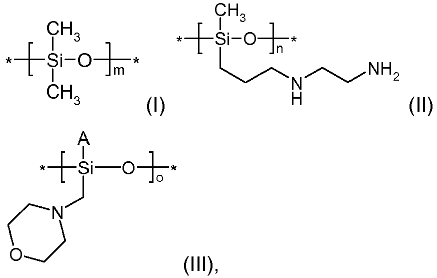 oder einen über ein -O- gebundenen oligomeren oder polymeren Rest enthaltend Struktureinheiten der Formeln (I), (II) oder (III) oder die Hälfte eines verbindenden O-Atoms zu einer Struktureinheit (III) oder für -OH steht,
* für eine Bindung zu einer der Struktureinheiten (I), (II) oder (III) oder für eine Endgruppe B (Si-gebunden) oder D (O-gebunden) steht,
B für eine Gruppe -OH, -O-Si(CH₃)₃,-O-Si(CH₃)₂OH, -O-Si(CH₃)₂OCH₃ steht,
D für eine Gruppe -H, -Si(CH₃)₃,-Si(CH₃)₂OH, -Si(CH₃)₂OCH₃ steht,
a, b und cfür ganze Zahlen zwischen 0 und 1000 stehen, mit der Maßgabe a + b + c > 0
m, n und o für ganze Zahlen zwischen 1 und 1000 stehen.

7. Mittel nach einem der Ansprüche 1 bis 6, **dadurch gekennzeichnet, dass** das/die Silikonöl(e) der Formel (I) mindestens eine endständige Dimethylsilylhydroxy-Gruppe aufweisen, d.h. ausgewählt sind aus Silikonen, in denen
| | | |
|---|---|---|
| B = -O-Si(CH₃)₂OH | und | D = -Si(CH₃)₃ |
| B = -O-Si(CH₃)₂OH | und | D = -Si(CH₃)₂OH |
| B = -O-Si(CH₃)₂OH | und | D = -Si(CH₃)₂OCH₃ |
| B = -O-Si(CH₃)₃ | und | D = -Si(CH₃)₂OH |
| B = -O-Si(CH₃)₂OCH₃ | und | D = -Si(CH₃)₂OH. |

8. Mittel nach einem der Ansprüche 1 bis 7, **dadurch gekennzeichnet, dass** es in der Zubereitung (A) Silikonöl(e) der Formel (I) enthält, bei dem/denen
- m > (n + o) bzw. (a+b+c) > (n + o), vorzugsweise
- m > 10(n + o) bzw. (a+b+c) > 10(n + o), besonders bevorzugt
- m > 50(n + o) bzw. (a+b+c) > 50(n + o), weiter bevorzugt
- m > 75(n + o) bzw. (a+b+c) > 75(n + o) und insbesondere
- m > 100(n + o) bzw. (a+b+c) > 100(n + o) gilt.

9. Mittel nach einem der Ansprüche 1 bis 8, **dadurch gekennzeichnet, dass** es- bezogen auf sein Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% verzweigtes, ethoxyliertes Tridecanol (INCI-Bezeichnung: Trideceth-5) oder α-iso-Tridecyl-ω-hydroxypolyglycolether (INCI-Bezeichnung: Trideceth-10) oder deren Mischungen enthält.

10. Mittel nach einem der Ansprüche 1 bis 9, **dadurch gekennzeichnet, dass** Zubereitung (A) frei von Xanthan ist und - bezogen auf ihr Gewicht - 5 bis 60 Gew.-%, vorzugsweise 10 bis 55 Gew.-%, besonders bevorzugt 15 bis 50 Gew.-% und insbesondere 20 bis 45 Gew.-% mindestens eines Persulfat, ausgewählt aus Ammoniumperoxodisulfat und/oder Kaliumperoxodisulfat und/oder Natriumperoxodisulfat, enthält.

11. Mittel nach einem der Ansprüche 1 bis 9, **dadurch gekennzeichnet, dass** die Zubereitung (B) - bezogen auf ihr Gewicht- 0,1 bis 10 Gew.-%, vorzugsweise 0,25 bis 7,5 Gew.-%, weiter bevorzugt 0,5 bis 5 Gew.-%, besonders bevorzugt 0,75 bis 4 Gew.-% und insbesondere 1 bis 2,5 Gew.-% Xanthan und 0,5 bis 30 Gew.-%, vorzugsweise 1 bis 20 Gew.-%, besonders bevorzugt 5 bis 15 Gew.-% und insbesondere 6 bis 12 Gew.-% Wasserstoffperoxid, berechnet als 100%iges H₂O₂, enthält.

12. Verfahren zur Farbveränderung keratinischer Fasern, bei dem mindestens zwei getrennt voneinander verpackte Zubereitungen (A) und (B), von welchen Zubereitung (A) mindestens ein Persulfat, mindestens ein Acrylatpolymer und Carboxymethylcellulose und Zubereitung (B) mindestens ein Oxidationsmittel enthält, zu einer Anwendungsmischung vermischt werden, diese auf die Fasern aufgetragen wird und nach einer Einwirkungszeit wieder abgespült wird, **dadurch gekennzeichnet, dass**
i. Zubereitung (A) - bezogen auf ihr Gewicht -
a1) 0 bis 3 Gew.-% Keratinhydrolysat(e) und/oder
a2) 0 bis 5 Gew.-% Silikonöl(e) enthält,
mit der Maßgabe, dass die Zubereitung (A) - bezogen auf ihr Gewicht - 0,1 bis 6 Gew.-% Verbindung(en) aus den Gruppen a1) und a2) enthält.

13. Verfahren nach Anspruch 9, **dadurch gekennzeichnet, dass** die Kontrolle des Aufhellgrades der keratinischen Faser während der Einwirkzeit ohne Entfernen der Anwendungsmischung von der Faser visuell erfolgt.

## Claims

1. An agent for lightening keratinic fibers, containing at least two preparations (A) and (B) packaged separately from one another, as well as optionally a further preparation (C) packaged separately from (A) and (B), which preparations are mixed immediately prior to application to form an application mixture,
i. preparation (A) containing at least one persulfate, at least one acrylate polymer and carboxymethyl cellulose, and
ii. preparation (B) being flowable and containing at least one oxidizing agent,
**characterized in that** preparation (A) contains, based on its weight,
a1) 0 to 3 wt.% keratin hydrolysate(s) and/or
a2) 0 to 5 wt.% silicone oil(s),
provided that preparation (A) contains, based on its weight, D.1 to 6 wt.% campound(s) from the groups a1) and a2).

2. The agent according to claim 1, **characterized in that** preparation (A) contains 0.01 to 1 wt.%, preferably 0.05 to 0.75 wt.%, more preferably 0.1 to 0.5 wt.%, particularly preferably 0.15 to 0.4 wt.% and in particular 0.2 to 0.3 wt.% hydrolysate(s) having molar masses of from 400 to 1200 daltons, obtained from the cortex and/or the cuticle of keratinic fibers.

3. The agent according to one of claims 1 or 2, **characterized in that** preparation (A) contains 0.01 to 5 wt.%, preferably 0.05 to 4 wt.%, more preferably 0.1 to 2.5 wt.%, particularly preferably 0.25 to 2 wt.% and in particular 0.5 to 1.5 wt.% silicone oil(s) from the group of compounds having the INCI name Dimethicone.

4. The agent according to one of claims 1 to 3, **characterized in that** preparation (B) contains, based on its weight, 0.01 to 5 wt.%, preferably 0.05 to 4 wt.%, more preferably 0.1 to 2.5 wt.%, particularly preferably 0.25 to 2 wt.% and in particular 0.5 to 1.5 wt.% cosmetic oil(s), preferably silicone oil(s).

5. The agent according to one of claims 1 to 4, **characterized in that** contains 0.01 to 5 wt.%, preferably 0.05 to 4 wt.%, more preferably 0.1 to 2.5 wt.%, particularly preferably 0.25 to 2 wt.% and in particular 0.5 to 1.5 wt.% silicone oil(s) from the group of compounds having the INCI name Amodimethicone, compounds having the INCI names Silicone Quaternium-1, Silicone Quaternium-2, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, Silicone Quaternium-16, Silicone Quaternium-17, Silicone Quaternium-18, Silicone Quaternium-20, Silicone Quaternium-21 or Silicone Quaternium-22 being preferred.

6. The agent according to one of claims 1 to 5, **characterized in that** preparation (A) contains 0.01 to 5 wt.%, preferably 0.05 to 4 wt.%, more preferably 0.1 to 2.5 wt.%, particularly preferably 0.25 to 2 wt.% and in particular 0.5 to 1.5 wt.% silicone oil(s) of formula (I), 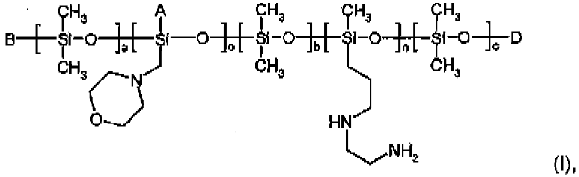 in which
A represents a structural unit (I), (II) or (III) bound by an -O- 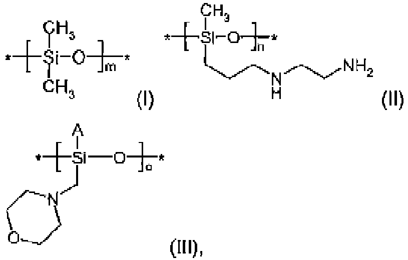 or an oligomeric or polymeric functional group bound by an -O- and containing structural units of formulas (I), (II) or (III) or half of a connecting O atom to a structural unit (III), or represents -OH,
represents a bond to one of the structural units (I), (II) or (III) or represents a terminal group B (Si-bound) or D (O-bound),
B represents a group -OH, -O-Si(CH₃)₃, -O-Si(CH₃)₂OH, -O-Si(CH₃)₂OCH₃,
D represents a group -H, -Si(CH₃)₃, -Si(CH₃)₂OH, -Si(CH₃)₂OCH₃,
a, b and c represent integers between 0 and 1000, provided that a + b + c > 0
m, n, and o represent integers between 1 and 1000.

7. The agent according to one of claims 1 to 6, **characterized in that** the silicone oil(s) of formula (I) has/have at least one terminal dimethylsilylhydroxy group, i.e. selected from silicones, in which
| | | |
|---|---|---|
| B = -O-Si(CH₃)₂OH | and | D = -Si(CH₃)₃ |
| B = -O-Si(CH₃)₂OH | and | D = -Si(CH₃)₂OH |
| B = -O-Si(CH₃)₂OH | and | D = -Si(CH₃)₂OCH₃ |
| B = -O-Si(CH₃)₃ | and | D = -Si(CH₃)₂OH |
| B = -O-Si(CH₃)₂OCH₃ | and | D = -Si(CH₃)₂OH. |

8. The agent according to one of claims 1 to 7, **characterized in that** it contains, in preparation (A), silicone oil(s) of formula (I), in which
- m > (n + o) or (a+b+c) > (n + o), preferably
- m > 10(n + o) or (a+b+c) > 10(n + o), particularly preferably
- m > 50(n + o) or (a+b+c) > 50(n + o), more preferably
- m > 75(n + o) or (a+b+c) > 75(n + o) and in particular
- m > 100(n + o) or (a+b+c) > 100(n + o) applies.

9. The agent according to one of claims 1 to 8, **characterized in that** it contains, based on its weight, 0,00001 to 5 wt.%, preferably 0.0001 to 3.5 wt.%, particularly preferably 0.001 to 2 wt.%, more preferably 0.01 to 1 wt.%, and in particular 0.1 to 0.5 wt.% branched ethoxylated tridecanol (INCI name: Trideceth-5) or α-isotridecyl-ω-hydroxypolyglycol ether (INCI name; Trideceth-10) or mixtures thereof.

10. The agent according to one of claims 1 to 9, **characterized in that** preparation (A) is free of xanthan and contains, based on its weight, 5 to 60 wt.%, preferably 10 to 55 wt.%, particularly preferably 15 to 50 wt.% and in particular 20 to 45 wt.% of at least one persulfate selected from the group consisting of ammonium peroxodisulfate and/or potassium peroxodisulfate and/or sodium peroxodisulfate.

11. The agent according to one of claims 1 to 9, **characterized in that** preparation (B) contains, based on its weight, 0.1 to 10 wt.%, preferably 0.25 to 7.5 wt.%, more preferably 0.5 to 5 wt.%, particularly preferably 0.75 to 4 wt.% and in particular 1 to 2.5 wt.% xanthan and 0.5 to 30 wt.%, preferably 1 to 20 wt.%, particularly preferably 5 to 15 wt.% and in particular 6 to 12 wt.% hydrogen peroxide, calculated as 100% H₂O₂.

12. A method for changing the color of keratinic fibers, in which at least two preparations (A) and (B) packaged separately from one another, of which preparation (A) contains at least one persulfate, at least one acrylate polymer, and carboxymethyl cellulose, and preparation (B) contains at least one oxidizing agent, are mixed to form an application mixture which is applied to the fibers and rinsed out after an exposure period,
**characterized in that**
i. preparation (A) contains, based on its weight,
a1) 0 to 3 wt.% keratin hydrolysate(s) and/or
a2) 0 to 5 wt.% silicone oil(s),
provided that preparation (A) contains, based on its weight, 0.1 to 6 wt.% compound(s) from the groups a1) and a2).

13. The method according to claim 9, **characterized in that** the degree of lightening of the keratinic fiber is visually checked during the exposure period without removing the application mixture from the fiber.

## Revendications

1. Produit d'éclaircissement de fibres de kératine contenant au moins deux préparations emballées séparément l'une de l'autre (A) et (B), ainsi qu'éventuellement une autre préparation (C) emballée séparément de (A) et (B), qu'on mélange immédiatement avant utilisation en un mélange d'utilisation, où :
i. la préparation (A) contient au moins un persulfate, au moins un polymère acrylate et une carboxyméthylcellulose, et
ii. la préparation (B) est fluide et contient au moins un oxydant,
**caractérisé en ce que** la préparation (A) contient, rapporté à son poids :
a1) 0 à 3 % en poids d'hydrolysat(s) de kératine, et/ou
a2) 0 à 5 % en poids d'huile(s) de silicone,
à condition que la préparation (A) contienne, rapporté à son poids, 0,1 à 6 % en poids de composé(s) des groupes a1) et a2).

2. Produit selon la revendication 1, **caractérisé en ce que** la préparation (A) contient 0,01 à 1 % en poids, de préférence 0,05 à 0,75 % en poids, plus préférentiellement 0,1 à 0,5 % en poids, particulièrement préférentiellement 0,15 à 0,4 % en poids et en particulier 0,2 à 0,3 % en poids d'hydrolysat(s) de masses molaires de 400 à 1200 Dalton récoltés dans le cortex et/ou la cuticule de fibres de kératine.

3. Produit selon la revendication 1 ou 2, **caractérisé en ce que** la préparation (A) contient 0,01 à 5 % en poids, de préférence 0,05 à 4 % en poids, plus préférentiellement 0,1 à 2,5 % en poids, particulièrement préférentiellement 0,25 à 2 % en poids et en particulier 0,5 à 1,5 % en poids d'huile(s) de silicone du groupe des composés de désignation INCI diméthicone.

4. Produit selon la revendication 1 à 3, **caractérisé en ce que** la préparation (B) contient, rapporté à son poids, 0,01 à 5 % en poids, de préférence 0,05 à 4 % en poids, plus préférentiellement 0,1 à 2,5 % en poids, particulièrement préférentiellement 0,25 à 2 % en poids et en particulier 0,5 à 1,5 % en poids d'huile(s) cosmétiques, de préférence d'huile(s) de silicone.

5. Produit selon la revendication 1 à 4, **caractérisé en ce qu'**il contient 0,01 à 5 % en poids, de préférence 0,05 à 4 % en poids, plus préférentiellement 0,1 à 2,5 % en poids, particulièrement préférentiellement 0,25 à 2 % en poids et en particulier 0,5 à 1,5 % en poids d'huile(s) de silicone du groupe des composés de désignation INCI amodiméthicone, où on préfère les composés de désignation INCI silicone Quaternium 1, silicone Quaternium 2, silicone Quaternium 3, silicone Quaternium 4, silicone Quaternium 5, silicone Quaternium 6, silicone Quaternium 7, silicone Quaternium 8, silicone Quaternium 9, silicone Quaternium 10, silicone Quaternium 11, silicone Quaternium 12, silicone Quaternium 15, silicone Quaternium 16, silicone Quaternium 17, silicone Quaternium 18, silicone Quaternium 20, silicone Quaternium 21 ou silicone Quaternium 22.

6. Produit selon la revendication 1 à 5, **caractérisé en ce que** la préparation (A) 0,01 à 5 % en poids, de préférence 0,05 à 4 % en poids, plus préférentiellement 0,1 à 2,5 % en poids, particulièrement préférentiellement 0,25 à 2 % en poids et en particulier 0,5 à 1,5 % en poids d'huile(s) de silicone de formule (I) : 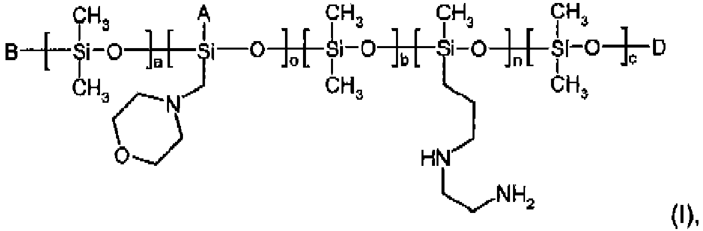 où
A représente une unité structurelle (I), (II) ou (III) liée à -O- : 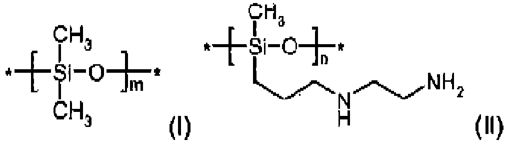 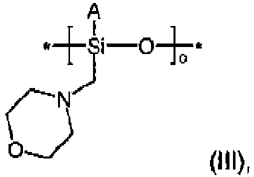 ou un résidu oligomère ou polymère lié à -O- contenant des unités structurelles de formule (I), (II) ou (III) ou la moitié d'un atome de liaison O appartient à une unité structurelle (III) ou représente -OH,
* représente une liaison à une des unités structurelles (I), (II) ou (III) ou un groupe terminal B (lié à Si) ou D (lié à O),
B représente un groupe -OH, -O-Si(CH₃)₃, -O-Si(CH₃)₂OH, -O-Si(CH₃)₂OCH₃,
D représente un groupe -H, -Si(CH₃)₃, -Si(CH₃)₂H, -Si(CH₃)₂OCH₃,
a, b et c représentent des entiers entre 0 et 1000, à conditions que a + b + c > 0
m, n et représentent des entiers entre 1 et 1000.

7. Produit selon la revendication 1 à 6, **caractérisé en ce que** le ou les huiles de silicone de formule (I) présentent au moins un groupe diméthylsilylhydroxy terminal, c'est à choisi parmi des silicones dans lesquels :
| | | |
|---|---|---|
| B = -O-Si(CH₃)₂OH | et | D = -Si(CH₃)₃ |
| B = -O-Si(CH₃)₂OH | et | D = -Si(CH₃)₂OH |
| B = -O-Si(CH₃)₂OH | et | D = -Si(CH₃)₂OCH₃ |
| B = -O-Si(CH₃)₃ | et | D = -Si(CH₃)₂OH |
| B = -O-Si(CH₃)₂OCH₃ | et | D = -Si(CH₃)₂OH. |

8. Produit selon la revendication 1 à 7, **caractérisé en ce qu'**il contient dans la préparation (A) une ou plusieurs huiles de silicone de formule (I), où
| | | | |
|---|---|---|---|
| - | m > (n + o) | ou resp. | (a + b + c) > (n + o), de préférence |
| - | m > 10 (n + o) | ou resp. | (a + b + c) > 10 (n + o), notamment |
| - | m > 50 (n + o) | ou resp. | (a + b + c) > 50 (n + o), plus |
| | préférentiellement | | |
| - | m > 75 (n + o) | ou resp. | (a + b + c) > 75 (n + o), et en particulier |
| - | m > 100 (n + o) | ou resp. | (a + b + c) > 100 (n + o). |

9. Produit selon la revendication 1 à 8, **caractérisé en ce qu'**il contient, rapporté à son poids, 0,00001 à 5 % en poids, de préférence 0,0001 à 3,5 % en poids, particulièrement préférentiellement 0,001 à 2 % en poids, plus préférentiellement 0,01 à 1 % en poids et en particulier 0,1 à 0,5 % en poids de Tridecanol éthoxylé ramifié (désignation INCI : Trideceth 5) ou α-iso-tridécyl-ω-hydroxypolyglycoléther (désignation INCI : Trideceth 10) ou leurs mélanges.

10. Produit selon la revendication 1 à 9, **caractérisé en ce que** la préparation (A) est exempte de xanthane et contient, rapporté à son poids, 5 à 60 % en poids, de préférence 10 à 55 % en poids, particulièrement préférentiellement 15 à 50 % en poids et en particulier 20 à 45 % en poids d'au moins un persulfate choisi parmi le peroxodisulfate d'ammonium et/ou le peroxodisulfate de potassium et/ou le peroxodisulfate de sodium.

11. Produit selon la revendication 1 à 9, **caractérisé en ce que** la préparation (B) contient 0,1 à 10 % en poids, de préférence 0,25 à 7,5 % en poids, plus préférentiellement 0,5 à 5 % en poids, particulièrement préférentiellement 0,75 à 4 % en poids et en particulier 1 à 2,5 % en poids de xanthane et 0,5 à 30 % en poids, de préférence 1 à 20 % en poids, particulièrement préférentiellement 5 à 15 % en poids et en particulier 6 à 12 % en poids de peroxyde d'hydrogène, calculé comme H₂O₂ à 100 %.

12. Procédé de modification de la couleur de fibres de kératine, dans lequel on mélange au moins deux préparations emballées séparément l'une de l'autre (A) et (B), dont la préparation (A) contient au moins un persulfate, au moins un polymère acrylate et une carboxyméthylcellulose, et la préparation (B) contient au moins un oxydant, en un mélange prêt à l'emploi qu'on applique sur les fibres, et qu'on rince après une durée d'action, **caractérisé en ce que** :
i. la préparation (A) contient rapporté à son poids :
a1) 0 à 3 % en poids d'hydrolysat(s) de kératine, et/ou
a2) 0 à 5 % en poids d'huile(s) de silicone,
à condition que la préparation (A) contienne, rapporté à son poids, 0,1 à 6 % en poids de composé(s) des groupes a1) et a2).

13. Procédé selon la revendication 9, **caractérisé en ce que** le contrôle du degré d'éclaircissement des fibres de kératine pendant la durée d'action se fait visuellement sans enlever le mélange prêt à l'emploi des fibres.
